# EUROPÄISCHE PATENTSCHRIFT

(11) **EP 2 644 220 B1**
(45) Veröffentlichungstag und Bekanntmachung des Hinweises auf die Patenterteilung: **31.05.2017**
(21) Anmeldenummer: 13001475.6
(22) Anmeldetag: 22.03.2013
(51) Int. Cl.: A61M 16/00

(54) **Vorrichtung zur Durchführung von Hyperinsufflationen**
Device for performing hyper-insufflations
Dispositif destinés à l'hyperinsufflation

(30) Priorität: 28.03.2012 DE 102012006159
(43) Veröffentlichungstag der Anmeldung: 02.10.2013
(73) Patentinhaber: Löwenstein Medical Technology S.A., 2557 Luxembourg (LU)
(72) Erfinder: Feldhahn, Karl-Andreas, 22761 Hamburg (DE); Göbel, Christof, 22457 Hamburg (DE)
(74) Vertreter: Marx, Thomas

(56) Entgegenhaltungen:
- DE-A1-102007 033 048
- DE-C1- 19 961 253
- US-A- 3 961 627
- US-A- 4 215 681
- US-A- 5 353 788
- US-A- 5 540 220
- US-A1- 2006 211 950
- US-A1- 2007 068 528
- US-A1- 2010 095 964
- US-A1- 2011 023 878

## Beschreibung

Die vorliegende Erfindung betrifft eine Vorrichtung zur Zuführung eines Atemgases die neben der konventionellen Beatmung, über die Option der Durchführung von Hyperinsufflationsmanövern verfügt. Hyperinsufflationen stellen vertiefte Beatmungshübe dar, die verschiedenen therapeutischen Zwecken dienen können. Dazu gehören beispielsweise die Therapie von Atelektasen, die Unterstützung eines nachfolgenden Hustenstoßes durch die Hyperinsufflation sowie die Dehnung des Thorax bei regelmäßiger und langfristiger Anwendung zur Aufrechterhaltung der Thoraxbeweglichkeit. Patienten mit neuromuskulären Erkrankungen oder Rückenmarkverletzungen haben typischerweise eine erheblich eingeschränkte Lungenfunktion und werden oft invasiv oder nichtinvasiv beatmet.

Das Abhusten von Bronchialsekreten fällt diesen Patienten aufgrund ihrer Erkrankung schwer. Aufgrund der Muskelschwäche ist eine zunächst notwendige tiefe Inspiration vor dem Hustenstoß erschwert oder unmöglich. gleichzeitig fehlt die für einen wirksamen Hustenstoß erforderliche exspiratorische Kraft.

Die DE 10 2007 033 048 A1 offenbart eine Vorrichtung zur Zuführung eines Atemgases mit einer Druckgasquelle, einer Ausgabeeinrichtung und einem Controller, der die Druckgasquelle veranlasst, ausgehend vom einem Basis-Druckniveau, zumindest ein erhöhtes Druckniveau bereitzustellen, wobei die Vorrichtung zudem eine Zähleinrichtung aufweist die vom Controller aktiviert wird eine vorgebbare Zeitdauer zu zählen. Der Controller der DE 10 2007 033 048 A1 ist ferner ausgebildet, die Ausgabeeinrichtung zur Ausgabe eines wahrnehmbaren Signals zu veranlassen, um den Anwender an die Durchführung eines Hustenmanövers zu erinnern. Die Aufgabe der vorliegenden Erfindung ist es, eine Vorrichtung und ein Verfahren zur Zuführung eines Atemgases anzugeben, dass das geräteseitige Druckmanöver durch charakteristische Merkmale des Druckverlaufes, einhergehend mit der Identifikation eines optimalen Zeitpunktes, in verbesserter Weise für ein Hustenmanöver geeignet ist und der Patient somit den Hustenstoß optimal mit dem Gerät koordinieren kann.

Die erfindungsgemäße Vorrichtung zur Zuführung eines Atemgases weist dazu eine Druckgasquelle, einer Ausgabeeinrichtung und einem Controller auf, der die Druckgasquelle veranlasst, ausgehend vom einem Basis-Druckniveau, zumindest ein erhöhtes Druckniveau bereitzustellen, wobei die Vorrichtung zudem eine Zähleinrichtung aufweist die vom Controller aktiviert wird wobei die Zähleinrichtung bei Erreichen eines Druckplateaus bei einem erhöhten Atemgasdruck vom Controller aktiviert wird eine vorgebbare Zeitdauer im Bereich 0,250 bis 4 Sekunden zu zählen und der Controller ausgebildet ist, die Ausgabeeinrichtung im Wesentlichen zeitgleich mit der Aktivierung der Zähleinrichtung zur Ausgabe eines wahrnehmbaren Signals zu veranlassen und die Druckgasquelle nach Ablauf der Zeitdauer vom Controller zur Absenkung des Atemgasdruckes veranlasst wird.

Bei dem erfindungsgemäßen Verfahren zur Zuführung eines Atemgases - welches nicht der chirurgischen oder therapeutischen Behandlung des menschlichen oder tierischen Körpers dient - veranlasst ein Controller eine Druckgasquelle, ausgehend vom einem Basis-Druckniveau , zumindest ein erhöhtes Druckniveau bereitzustellen, wobei der Controller zudem eine Zähleinrichtung und eine Ausgabeeinrichtung aktiviert wobei der Controller bei Erreichen eines Druckplateaus bei einem erhöhten Atemgasdruck eine Zähleinrichtung aktiviert eine vorgebbare Zeitdauer im Bereich 0,25 bis 4 Sekunden zu zählen, wobei der Controller nach Ablauf dieser Zeitdauer die Druckgasquelle zur Absenkung des Atemgasdruckes veranlasst, und wobei der Controller eine Ausgabeeinrichtung zur Ausgabe eines wahrnehmbaren Signals veranlasst, wobei der Controller die Ausgabeeinrichtung im Wesentlichen zeitgleich mit der Aktivierung der Zähleinrichtung aktiviert.

Die erfindungsgemäße Vorrichtung unterstützt diese Patienten beim Abhusten dadurch, dass ausgehend vom Einatmungsdruck (IPAP) ein erhöhter Atemgasdruck bereitgestellt wird, wodurch eine gegenüber der normalen Beatmung tiefere Einatmung bewirkt und Lunge und Thorax vorgespannt werden. Für die Durchführung des Hustenstoßes ist patientenseitig eine gute Koordination mit einem geräteseitigen Druckmanöver notwendig. Hierzu muss der Patient im richtigen Moment vor dem Wiederabfall des Druckes seine Stimmritze (Glottis) schließen, um für einen Moment die Luft in der Lunge zu halten.

Durch ein vorrichtungsseitig generiertes Signal, welches bei Erreichen eines Druckplateaus bei einem erhöhten Atemgasdruck ertönt, kann der Patient den besten Zeitpunkt für den Schluss der Glottis bestimmen und sich optimal mit dem Gerät synchronisieren sowie im Anschluss selbst aktiv einen Hustenstoß durchführen. Gerätetechnisch wird ein optimaler Husten durch das schnelle Absenken des Druckes im Anschluss an die Insufflation unterstützt.

Die erfindungsgemäße Vorrichtung zeichnet sich vorteilhaft dadurch aus, dass die vorgebbare Zeitdauer im Bereich 0,250 bis 4 Sekunden ist. Diese Zeitdauer bietet dem Patienten ausreichend Zeit, einen Hustenstoß durch Schließen der Glottis vorzubereiten.

Alternativ ist vorgesehen, dass die minimale Zeitdauer für die Aufrechterhaltung des maximalen Druckniveaus 0,5 s ist und die maximale Zeitdauer 2 s ist.

Eine Optimierung der Gerätebetriebsweise kann dadurch erfolgen, dass der Controller die Ausgabeeinrichtung im Wesentlichen zeitgleich mit der Aktivierung der Zähleinrichtung aktiviert. Dies führt dazu, dass das Signal zu Beginn des maximalen Druckniveaus erzeugt wird und der Patient entsprechend frühzeitig informiert ist.

Eine Individualisierung der Gerätebetriebsweise kann dadurch erfolgen, dass die Vorrichtung eine Sensorik aufweist die geeignet ist das Triggersignal eines Patienten/Anwenders zu registrieren, woraufhin der Controller die Druckgasquelle veranlasst ein erhöhtes Druckniveau bereitzustellen.

Eine Optimierung der Gerätebetriebsweise kann auch dadurch erfolgen, dass die Druckgasquelle das maximale Druckniveau im Wesentlichen aufrecht erhält (= Plateau).

Gemäß einer typischen Gerätebetriebsweise ist vorgesehen, dass das Basis-Druckniveau ein exspiratorisches Druckniveau ist oder 0 hPa beträgt.

Eine effektive Gerätebetriebsweise kann dadurch erfolgen, dass das erhöhte, maximale Druckniveau zumindest 10% über dem Wert des inspiratorischen Beatmungsdruckes (IPAP) liegt.

Eine Automatisierung der Gerätebetriebsweise kann dadurch erfolgen, dass das erhöhte Druckniveau nach Ablauf der maximalen Zeitdauer auf eine Ausatmungs-Druckniveau abgesenkt wird.

Eine weitere Individualisierung der Gerätebetriebsweise kann dadurch erfolgen, dass die Charakteristik der Druckanhebung auf das erhöhte Druckniveau einstellbar ist.

Eine weitere Individualisierung der Gerätebetriebsweise auf patientenspezifische Bedarfe kann dadurch erfolgen, dass die Charakteristik der Druckabsenkung vom erhöhten Druckniveau auf das Basisdruckniveau einstellbar ist.

Ein Hustenstoß wird optimal unterstützt indem die Charakteristik der Druckanhebung durch die Vorrichtung immer von der Charakteristik der Druckabsenkung abweicht, wobei die Druckabsenkung zumindest phasenweise schneller erfolgt als die Druckanhebung.

In diesem Zusammenhang ist auch vorgesehen, dass die Charakteristik der Druckanhebung und/oder Druckabsenkung rampenförmig ist.

Zusätzlich kann vorgesehen werden, dass die Druckanhebung und/oder Druckabsenkung mit gleichmäßiger und/oder veränderlicher Rampensteigung erfolgt.

Die erfindungsgemäße Vorrichtung kann ebenfalls dazu genutzt werden, Patienten einen Atemgashub mit erhöhtem Atemgasdruck, im Sinne eines Seufzers, bereitzustellen, wobei beispielsweise die Bekämpfung von Atelektasen oder langfristig die Aufrechterhaltung der Thoraxbeweglichkeit im Vordergrund steht, die bei Patienten beispielsweise mit neuromuskulären Erkrankungen in der Regel zunehmend schlechter wird.

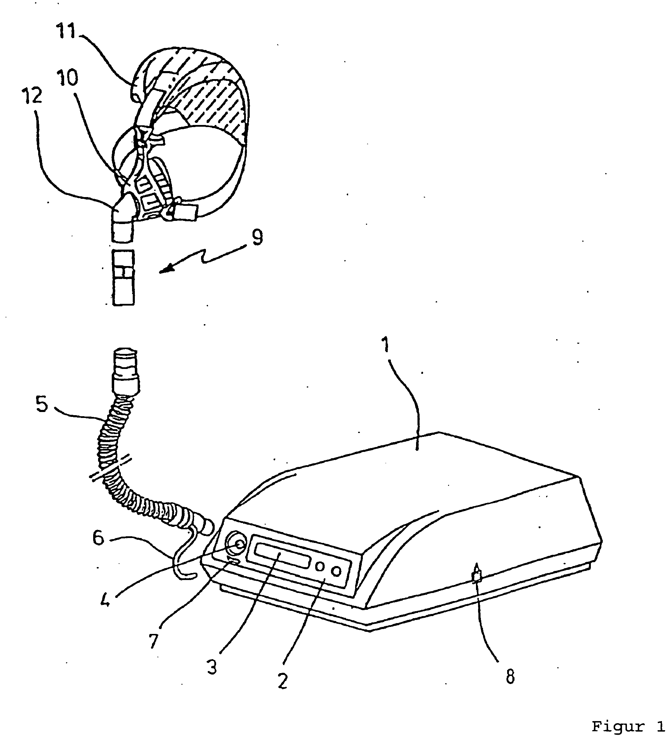

Figur 1 zeigt den grundsätzlichen Aufbau einer Vorrichtung zur Zuführung eines Atemgases. Im Bereich eines Gerätegehäuses (1) mit Bedienfeld (2) sowie Anzeige (3) ist in einem Geräteinnenraum eine Druckgasquelle (13) angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden. In diesem Fall verfügt das gezeigte Gerätegehäuse über einen weiteren nicht dargestellten Anschluss zur Ansteuerung des Ausatemventils. Figur 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienteninterface (10) ein Kupplungselement (12) auf.

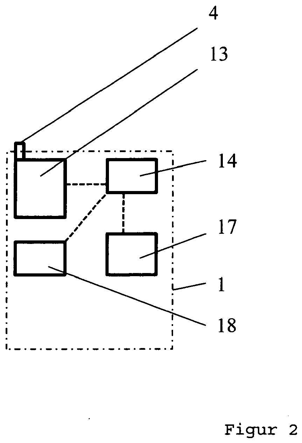

Figur 2 zeigt eine Vorrichtung zur Zuführung eines Atemgases mit einer Druckgasquelle (13) und einem Controller (14), der die Druckgasquelle (13) veranlasst, ausgehend vom einem Basis-Druckniveau (15), zumindest ein erhöhtes Druckniveau (16, 26) bereitzustellen. Die Vorrichtung weist zudem eine Zähleinrichtung (17) auf, die mit dem Übergang auf das maximale Druckniveau vom Controller (14) aktiviert wird und dann eine vorgebbare Zeitdauer im Bereich 0,250 bis 4 Sekunden, bevorzugt 0,5 bis 2 Sekunden, zählt. Während der Zeitdauer erhält die Druckgasquelle das Druckniveau im Wesentlichen aufrecht. Nach Ablauf dieser Zeitdauer wird die Druckgasquelle (13) vom Controller (14) zur Absenkung des Atemgasdruckes veranlasst.

Der Controller (14) ist ferner ausgebildet, eine Ausgabeeinrichtung (18) zur Ausgabe eines wahrnehmbaren Signals (22) zu veranlassen. Die Ausgabeeinrichtung ist bevorzugt ein Lautsprecher, welcher ein kurzes Tonsignal (22) ausgibt. Denkbar ist auch alternativ oder ergänzend zum Tonsignal ein optisches Signal. Bevorzugt aktiviert der Controller (14) die Ausgabeeinrichtung (18) im Wesentlichen zeitgleich mit der Aktivierung der Zähleinrichtung (17).

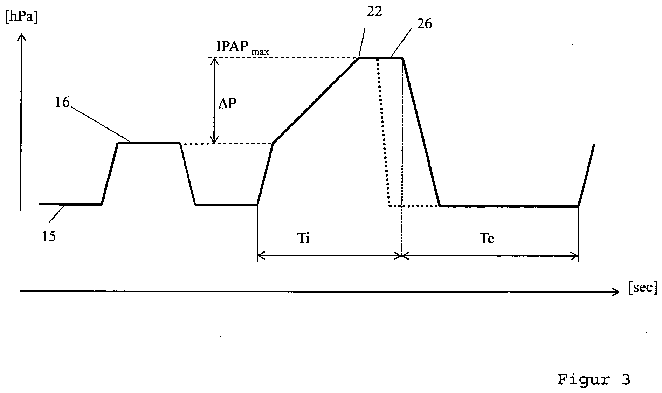

Figur 3 zeigt einen typischen Druckverlauf der erfindungsgemäßen Vorrichtung. Ausgehend von einem Basis-Druckniveau (15), welches bei 0 hPa liegt oder alternativ einen geringen positiven Druckwert, beispielsweise 1 - 5 hPa, annehmen kann, veranlasst der Controller (14) die Druckgasquelle (13) zumindest ein erhöhtes Druckniveau (16, 26) einzustellen. Das erste erhöhte Druckniveau (16) stellt bei einer Anwendung der Erfindung im Rahmen einer Beatmung ein Einatmungsdruckniveau (IPAP) dar. Die erfindungsgemäße Vorrichtung kann auf Anwenderauswahl hin oder durch einen Triggerimpuls aktiviert - auch ohne vorherige Zwischenstufe (IPAP) - das erhöhte Druckniveau (26) einstellen. Das Triggersignal wird bevorzugt durch einen Patienten ausgelöst, der über ein Mundstück (10) oder ein anderes Patienteninterface (10) einen Atemgashub abgibt. Die Druck-und/oder Fluss-Sensorik der Vorrichtung registriert diesen Atemgashub als Trigger, woraufhin der Controller (14) die Druckgasquelle (13) veranlasst ein erhöhtes Druckniveau bereitzustellen. Erfindungsgemäß ist auch vorgesehen, die Vorrichtung durch einen Patienten- oder Anwendertrigger zu aktivieren, woraufhin der Controller (14), die Druckgasquelle (13) veranlasst, ausgehend vom einem Basis-Druckniveau (15), sofort ein erhöhtes Druckniveau (26) einzustellen.
Die Charakteristik der Druckanhebung ist einstellbar und erfolgt bevorzugt rampenförmig. Die Druckanhebung kann unter einer gleichmäßigen Rampensteigung auf das erhöhte Druckniveau stattfindet. Die Druckanhebung kann mit einer veränderlichen Rampensteigung erfolgen. Das erhöhte Druckniveau (26) liegt zumindest 10% über dem Wert des erhöhten Druckniveaus (16) (IPAP = Einatmungsdruck) für die grundlegende Beatmung. Delta P liegt bei zumindest 1,5 hPa. Bei Erreichen des erhöhten Druckniveaus (26) gibt die Signaleinrichtung (18) ein Tonsignal (22) ab. Dieses dient dazu, dem Patienten zu signalisieren, dass er einen Hustenstoß vorbereiten kann. Der Wert des erhöhten Druckniveaus (26) ist bevorzugt in hPa Schritten oder Bruchteilen von hPa einstellbar. Die Druckgasquelle (13) hält das maximale Druckniveau (26) für 0,250 bis 4 Sekunden im Wesentlichen aufrecht (= Plateau (20)). Alternativ kann der Druck hier leicht ansteigend (0,1 - 1 hPa/sec) oder leicht abfallend gehalten werden. Nach Ablauf der Zeitspanne veranlasst der Controller (14) die Druckgasquelle (13) zur Absenkung des Atemgasdruckes auf ein Ausatmungs-Druckniveau (15). Das Ausatmungs-Druckniveau ist gleich 0 mbar und kann alternativ einen geringen positiven Druckwert, beispielsweise 2 - 5 mbar, annehmen. Die Charakteristik der Druckabsenkung ist einstellbar. Die Druckabsenkung kann rampenförmig erfolgen. Die Druckabsenkung erfolgt bevorzugt stark abfallend im Bereich 1 - 20 hPa/sec. Erfindungsgemäß erfolgt der Druckanstieg zum maximalen Druckniveau (26) immer langsamer, als der Druckabfall vom Druckniveau (26) auf das Ausatemniveau; dieser erfolgt bevorzugt mit einem Druckabfall im Bereich 10 - 20 hPa/sec.
Der patientenseitige Hustenstoß wird durch die schnelle Druckabsenkung optimal unterstützt.

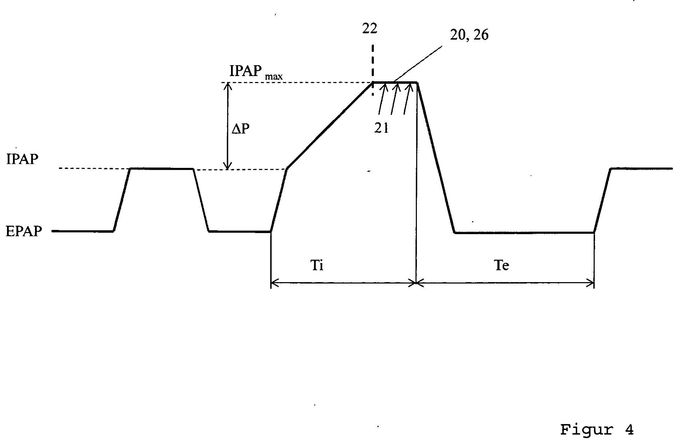

Figur 4 veranschaulicht, dass auf dem maximalen Druckniveau (26) der ideale Zeitraum, von 0,25 bis 4 Sekunden, für den patientenseitigen Glottisschluss (21) besteht, um den Hustenstoß optimal vorzubereiten. Gemäß der vorliegenden Erfindung bleibt dem Patienten im Zeitbereich den die Plateauphase andauert, somit genügend Zeit für einen Glottisschluss (21) und für die Vorbereitung auf den Hustenstoß.

Die Vorrichtung eignet sich ebenso zur Durchführung eines Hyperinsufflationsmanövers. Dazu weist sie eine Druckgasquelle (13) und einen Controller (14) auf, der die Druckgasquelle (13) veranlasst, ausgehend vom einem Basis-Druckniveau oder einem ersten erhöhten Druckniveau (16), auf ein Triggersignal hin, ein erhöhtes Druckniveau (26) bereitzustellen. Das Triggersignal kann durch einen Patienten ausgelöst werden, der über ein Mundstück oder ein anderes Patienteninterface (Nasalmaske, Mund-Nasenmaske, Tracheostoma, o.a.) einen Atemgashub abgibt. Die Druck- und/oder Fluss-Sensorik der Vorrichtung zu Beatmung registriert diesen Atemgashub als Trigger, woraufhin der Controller (14) die Druckgasquelle (13) veranlasst ein erhöhtes Druckniveau bereitzustellen. Die Aktivierung des Hyperinsufflationsmanövers kann auch zeitgesteuert erfolgen, wenn dieses im Rahmen einer Beatmung stattfindet oder vom Anwender abgerufen wird. Die Vorrichtung weist zudem eine Zähleinrichtung (17) auf, die mit dem Übergang auf das maximale Druckniveau zu zählen beginnt und eine vorgebbare Zeitdauer im Bereich 0,250 bis 4 Sekunden zählt, woraufhin der Controller die Druckgasquelle zur Absenkung des Atemgasdruckes veranlasst. Figur 3 veranschaulicht die durch das Hyperinsufflationsmanöver vertiefte Einatmung (Ti), bei der über einen erhöhten Druck auch ein größeres Atemgasvolumen appliziert wird. Entsprechend ist die Ausatemphase (Te) verlängert.

## Patentansprüche

1. Vorrichtung zur Zuführung eines Atemgases mit einer Druckgasquelle (13), einer Ausgabeeinrichtung (18) und einem Controller (14), der die Druckgasquelle (13) veranlasst, ausgehend vom einem Basis-Druckniveau (15), zumindest ein erhöhtes Druckniveau (16, 26) bereitzustellen, wobei die Vorrichtung zudem eine Zähleinrichtung (17) aufweist die vom Controller aktiviert wird **dadurch gekennzeichnet, dass** die Zähleinrichtung (17) bei Erreichen eines Druckplateaus bei einem erhöhten Atemgasdruck vom Controller (14) aktiviert wird eine vorgebbare Zeitdauer im Bereich 0,250 bis 4 Sekunden zu zählen und der Controller (14) ausgebildet ist, die Ausgabeeinrichtung (18) im Wesentlichen zeitgleich mit der Aktivierung der Zähleinrichtung (17) zur Ausgabe eines wahrnehmbaren Signals (22) zu veranlassen und die Druckgasquelle (13) nach Ablauf der Zeitdauer vom Controller (14) zur Absenkung des Atemgasdruckes veranlasst wird.

2. Vorrichtung nach Anspruch 1 **dadurch gekennzeichnet, dass** die Vorrichtung eine Sensorik aufweist die geeignet ist das Triggersignal eines Patienten/Anwenders zu registrieren, woraufhin der Controller (14) die Druckgasquelle (13) veranlasst ein erhöhtes Druckniveau (26) bereitzustellen.

3. Vorrichtung nach zumindest einem der vorhergehenden Ansprüche **dadurch gekennzeichnet, dass** die Druckgasquelle (13) das maximale Druckniveau (16, 26) im Wesentlichen aufrecht erhält (= Plateau) (20).

4. Vorrichtung nach zumindest einem der vorhergehenden Ansprüche **dadurch gekennzeichnet, dass** die minimale Zeitdauer für die Aufrechterhaltung des maximalen Druckniveaus (26) 0,5 s ist und die maximale Zeitdauer 2 s ist.

5. Vorrichtung nach zumindest einem der vorhergehenden Ansprüche **dadurch gekennzeichnet, dass** das Basis-Druckniveau (15) ein exspiratorisches Druckniveau ist oder 0 hPa beträgt.

6. Vorrichtung nach zumindest einem der vorhergehenden Ansprüche **dadurch gekennzeichnet, dass** das erhöhte Druckniveau (26) zumindest 10% über dem Wert des inspiratorischen Beatmungsdruckes (16) (IPAP) liegt.

7. Vorrichtung nach zumindest einem der vorhergehenden Ansprüche **dadurch gekennzeichnet, dass** das erhöhte Druckniveau (26) nach Ablauf der maximalen Zeitdauer auf eine Ausatmungs-Druckniveau (15) abgesenkt wird.

8. Vorrichtung nach zumindest einem der vorhergehenden Ansprüche **dadurch gekennzeichnet, dass** die Charakteristik der Druckanhebung auf das erhöhte Druckniveau (26) einstellbar ist.

9. Vorrichtung nach zumindest einem der vorhergehenden Ansprüche **dadurch gekennzeichnet, dass** die Charakteristik der Druckabsenkung vom erhöhten Druckniveau (26) auf das Basisdruckniveau einstellbar ist.

10. Vorrichtung nach zumindest einem der vorhergehenden Ansprüche **dadurch gekennzeichnet, dass** die Charakteristik der Druckanhebung immer von der Charakteristik der Druckabsenkung abweicht, wobei die Druckabsenkung zumindest phasenweise schneller erfolgt als die Druckanhebung.

11. Vorrichtung nach zumindest einem der vorhergehenden Ansprüche **dadurch gekennzeichnet, dass** die Druckanhebung und/oder Druckabsenkung mit gleichmäßiger und/oder veränderlicher Rampensteigung erfolgt.

## Claims

1. Device for supplying breathing gas with a compressed gas source (13), an output device (18), and a controller (14) that causes the compressed gas source (13), originating from a basic pressure level (15), to provide at least one increased pressure level (16, 26), wherein the device also comprises a counter (17) that is activated by the controller (14) **characterized in that** the counter (17) is activated by the controller when a pressure plateau is reached at an increased breathing gas pressure to count for a definable time period in a range from 0.250 to 4 seconds and the controller (14) is designed to cause the output device (18) to output a discernible signal (22) essentially at the same time as the activation of the counter (17) and the controller (14) causes the compressed gas source (13) to lower the breathing gas pressure after the time period has elapsed.

2. Device according to claim 1, **characterized in that** the device has sensors that are capable of registering the trigger signal of a patient/user, whereupon the controller (14) causes the compressed gas source (13) to provide an increased pressure level (26).

3. Device according to at least one of the preceding claims **characterized in that** the compressed gas source (13) essentially maintains the maximum pressure level (16, 26) (= plateau) (20).

4. Device according to at least one of the preceding claims **characterized in that** the minium time period for maintaining the maximum pressure level (26) is 0.5 s and the maximum time period is 2 s.

5. Device according to at least one of the preceding claims **characterized in that** basic pressure level (15) is an expiratory pressure level or 0 hPa.

6. Device according to at least one of the preceding claims **characterized in that** the increased pressure level (26) is at least 10% above the value of the inspiratory breathing pressure (16) (IPAP).

7. Device according to at least one of the preceding claims **characterized in that** the increased pressure level (26) is reduced to an exhalation pressure level (15) after the maximum time period.

8. Device according to at least one of the preceding claims **characterized in that** the characteristic of the pressure rise can be set/adjusted to the increased pressure level (26).

9. Device according to at least one of the preceding claims **characterized in that** the characteristic of the drop in pressure from the increased pressure level (26) to the basic pressure level can be set/adjusted.

10. Device according to at least one of the preceding claims **characterized in that** the characteristic of the pressure rise always deviates from the characteristic of the drop in pressure, where the drop in pressure occurs' faster than the pressure rise, at least at times.

11. Device according to at least one of the preceding claims **characterized in that** the pressure rise and/or drop in pressure occurs with an equal and/or variable ramp slope.

## Revendications

1. Dispositif destiné à délivrer un gaz respiratoire par le biais d'une source de gaz comprimé (13), d'un dispositif de sortie (18) et d'un contrôleur (14) qui permet à la source de gaz comprimé (13) de délivrer, en partant d'un niveau de pression de base (15), au moins un niveau de pression augmenté (16, 26), le dispositif présentant en outre un compteur (17) activé par le contrôleur et **caractérisé en ce qu'**il (17) est activé par le contrôleur (14) à compter durant une période définissable située entre 0,250 et 4 secondes lorsqu'un plateau de pression est atteint en présence d'une pression de gaz respiratoire accrue et **en ce que** le contrôleur (14) est conçu pour permettre au dispositif de sortie (18) de transmettre un signal (22) perceptible quasiment simultanément avec l'activation du compteur (17) et **en ce que** la source de gaz comprimé (13) est pilotée par le contrôleur (14) pour réduire la pression de gaz respiratoire après écoulement de ladite période.

2. Dispositif selon la revendication 1 **caractérisé en ce qu'**il intègre des capteurs appropriés pour enregistrer le signal de déclenchement d'un patient/utilisateur, à la suite de quoi le contrôleur (14) entraîne la source de gaz comprimé (13) à délivrer un niveau de pression (26) accru.

3. Dispositif selon au moins l'une des revendications précédentes **caractérisé en ce que** la source de gaz comprimé (13) maintient en grande partie le niveau de pression maximal (16, 26) (= plateau) (20).

4. Dispositif selon au moins l'une des revendications précédentes **caractérisé en ce que** la durée minimale de maintien du niveau de pression maximal (26) est de 0,5 seconde et la durée maximale, de 2 secondes.

5. Dispositif selon au moins l'une des revendications précédentes **caractérisé en ce que** le niveau de pression de base (15) est un niveau de pression expiratoire ou s'élève à 0 hPa.

6. Dispositif selon au moins l'une des revendications précédentes **caractérisé en ce que** le niveau de pression augmenté (26) se situe au moins 10 % au-dessus de la valeur de la pression de ventilation inspiratoire (16) (IPAP).

7. Dispositif selon au moins l'une des revendications précédentes **caractérisé en ce que** le niveau de pression augmenté (26) est réduit après écoulement de la durée maximale à un niveau de pression expiratoire (15).

8. Dispositif selon au moins l'une des revendications précédentes **caractérisé en ce que** la caractéristique de l'augmentation de pression puisse être réglée sur le niveau de pression augmenté (26).

9. Dispositif selon au moins l'une des revendications précédentes **caractérisé en ce que** la caractéristique de la diminution de pression à partir du niveau de pression augmenté (26) pour atteindre la niveau de pression de base est réglable.

10. Dispositif selon au moins l'une des revendications précédentes **caractérisé en ce que** la caractéristique de l'augmentation de pression diffère toujours de la caractéristique de la diminution de pression, la diminution de la pression étant au moins graduellement plus rapide que l'augmentation de la pression.

11. Dispositif selon au moins l'une des revendications précédentes **caractérisé en ce que** l'augmentation de la pression et/ou la diminution de la pression s'opèrent selon une inclinaison de rampe régulière et/ou variable.
